# EUROPEAN PATENT APPLICATION

(11) **EP 1 445 311 A1**
(43) Date of publication of application: **11.08.2004**
(21) Application number: 02802244.0
(22) Date of filing: 30.10.2002
(51) Int. Cl.: C12N 5/08

(54) **METHOD OF AMPLYFYING HEMATOPOIETIC STEM CELLS**

(30) Priority: 30.10.2001 JP 2001331940
(71) Applicant: DAIICHI PHARMACEUTICAL CO., LTD., Chuo-ku, Tokyo 103-8234 (JP)
(72) Inventor: NAWA, Katsuhiko, Daiichi Pharm. Co., Ltd., Tokyo 134-8630 (JP); HASUMURA, Mai, Daiichi Pharmaceutical Co., Ltd., Tokyo 134-8630 (JP); IMADA, Chiharu, Daiichi Pharmaceutical Co., Ltd, Tokyo 134-8630 (JP)
(74) Representative: Reitstötter - Kinzebach
(86) International application number: PCT/JP2002/011317
(87) International publication number: WO 2003/038077

(57) **Abstract**

A method for culturing Lin-negative or weakly-positive cells in the presence of macrophage colony-stimulating factor (M-CSF); a method for expanding hematopoietic stem cells by culturing Lin-negative or weakly-positive cells in the presence of M-CSF; hematopoietic stem cell populations obtained by the expanding method; a M-CSF-containing kit used for culturing Lin-negative or weakly-positive cells; and a M-CSF-containing agent used for expanding hematopoietic stem cells.

## Description

### TECHNICAL FIELD

The present invention relates to a method for expanding hematopoietic stem cells with macrophage colony-stimulating factor (M-CSF). More precisely, the invention relates to a method for culturing Lin-negative or weakly-positive cells in the presence of M-CSF, to a method for expanding hematopoietic stem cells by culturing Lin-negative or weakly-positive cells in the presence of M-CSF, to hematopoietic stem cell populations obtained by the expanding method, to a kit used for culturing Lin-negative or weakly-positive cells containing M-CSF, and to an agent used for expanding hematopoietic stem cells containing M-CSF.

### BACKGROUND ART

Hematopoietic stem cells are defined as cells that have both the ability to produce all hemocytes such as erythrocytes, leukocytes, platelets, T and B lymphocytes (pluripotency) and the ability to self-renew themselves (self-renewal capability). In bone marrow transplantation, the hematopoietic stem cells in the transplantedbonemarrow take a leading part, and therefore bone marrow transplantation may be reworded as hematopoietic stem cell transplantation. At present, bone marrow transplantation has been established as radical treatment for many intractable diseases such as variousblood diseases,cancers, immunodeficiency, congenital dysbolism. In such transplantation, however, the human leukocyte antigen (HLA) of the donor must be the same as that of the recipient, and a donor shortage is now an object of public concern.

Peripheral blood is often used these days as a source of hematopoietic stem cells in place of bone marrow. Only a small amount of CD34-positive cells including hematopoietic stem cells may exist in the peripheral blood of a healthy body, in which, however, a lot of such cells may be mobilized into the peripheral blood from the bone marrow thereof when granulocyte colony-stimulating factor (G-CSF) is continuously administered to it. Using a blood component separator, a lot of peripheral hematopoietic stem cells/hematopoietic precursor cells may be collected from the peripheral blood in this stage, and may be used in transplantation. However, this has some problems to be solved in point of the efficiency in mobilization of hematopoietic stem cells to peripheral blood and of the donor safety.

As another source of hematopoietic stem cells, cord blood may be used for cord blood stem cell transplantation. However, since the number of hematopoietic stem cells in cord blood is small, and such cord blood stem cell transplantation is now limited to children.

In that background, a technique is desired for *ex-vivo* expanding hematopoietic stem cells, for which various methods have heretofore been tried. For example, for expanding hematopoietic stem cells, reports have been announced that show the result of cell culture in the presence of cytokine such as stem cell factor (SCF), interleukin-3 (IL-3), interleukin-6 (IL-6), interleukin-11 (IL-11), G-CSF, granulocyte-macrophage colony-stimulating factor (GM-CSF) , fms-like tyrosine kinase-3 (Flt-3) ligand (FL), thrombopoetin (TPO). Hematopoietic precursor cells and cells differentiated from them may be expanded to a high degree when they are cultured along with such cytokines or any other growth factors, but the degree of expansion of hematopoietic stem cells cultured in that manner is only a few times and is not on a satisfactory level (Miller et al., *Proc. Natl. Acad. Sci*. *USA,* 94, 13648-13653, 1997; Matsunaga et al., *Blood,* 92, 452-461, 1998; Bryder et al., *Blood,* 96, 1748-1755, 2000). Hematopoietic precursor cells are generally defined as cells that have an *in-vitro* colony-forming ability. However, since they do not self-renew and since they cannot produce blood cells for a long period of time, they are useless as a source of hemocytes for transplantation.

In addition, a technique of expanding hematopoietic stem cells in co-culture with marrow stromal cells has been tried, in which, however, the degree of cell expansion is still only a few times (Moore et al., *Blood,* 89, 4337-4347, 1997).

On the other hand, if cytokine, growth factor or the like could be directly administered to living bodies to thereby significantly expand hematopoietic stem cells therein, then, for example, cord blood or the like that contains only a small amount of hematopoietic stem cells may be used as a transplantation source. However, no one has heretofore reported a successful experiment relating to it.

An object of the present invention is to realize a clinical application of hematopoietic stem cells expanded *ex-vivo* by increasing the level of expansion of hematopoietic stem cells with cytokines or growth factors *ex-vivo,* which has heretofore been insufficient. Another object is to expand hematopoietic stem cells *in-vivo* through direct cytokine administration to living bodies.

### DISCLOSURE OF THE INVENTION

As a result of extensive investigation to solve the above-mentioned problems, the present inventors have found that, when cells which are negative or weakly positive to a lineage marker (Lin) and which may contain hematopoietic stem cells are stimulated by macrophage colony-stimulating factor (M-CSF) or any other cytokine, then the expansion level of hematopoietic stem cells can be increased, thus completing the present invention.

That is, the invention relates to a method for culturing Lin-negative or weakly-positive cells in the presence of macrophage colony-stimulating factor (M-CSF).

Also, the invention relates to a method for expanding hematopoietic stem cells by culturing Lin-negative or weakly-positive cells in the presence of macrophage colony-stimulating factor (M-CSF).

Further, the invention relates to hematopoietic stem cell populations obtained by the above-mentioned expanding method.

Also, the invention relates to a kit used for culturing Lin-negative or weakly-positive cells containing macrophage colony-stimulating factor (M-CSF).

Further, the invention relates to an agent used for enhancing expansion of hematopoietic stem cells containing macrophage colony-stimulating factor (M-CSF).

### MODE FOR CARRYING OUT THE INVENTION

The method of culture and the method of expansion of the invention are characterized in that Lin-negative or weakly-positive cells are cultured in the presence of M-CSF. The present inventors cultured Lin-negative or weakly-positive cells in the presence of M-CSF to investigate as to whether or not hematopoietic stem cells could be expanded. As a result, we have found that the cell culture in the presence of M-CSF results in significant expansion of hematopoietic cells as compared with that in the absence of M-CSF. Accordingly, the method of culture of the invention is applicable to expansion of hematopoietic stem cells. Further, the hematopoietic stem cell populations expanded according to the expansion method of the invention may be used as a source for hematopoietic stem cell transplantation.

Preferably, Lin-negative or weakly-positive cells are collected based on a cell surface marker serving as an index to Lin. Lin means "lineage marker", and this includes, for example, an erythrocyte marker, Ter119; a granulocyte marker, Gr-1; a monocyte·macrophage marker Mac-1 (CD11b); and a T-cell marker Ly1. It is known that hematopoietic stem cells exist in Lin-negative or weakly-positive fractions.

Lin-negative or weakly-positive cells may be collected by treating a single cell suspension of marrow cells with labeled antibodies recognizing Lin, and removing the labeled cells by the use of cell sorter such as flow cytometer. Regarding mouse cells, for example, those processed with StemSep™ (StemCell Technologies Inc.) that contains an antibody cocktail for preparation of hematopoietic precursor cells are widely used for Lin-negative or weakly-positive cells. In the Example, the cells obtained by the use of StemSep™ were about 0.5% of the non-processed cells.

Thus obtained, the cells are cultured in the presence of M-CSF, for which the culture condition including the medium, the culture period and the culture plate is not specifically defined, but is suitably so selected that the cells can be kept good in the selected condition. In the Example, for example, the Lin-negative or weakly-positive cells prepared were seeded in a 6-well microplate for tissue culture (Iwaki) in an amount of 4 × 10⁴ cells/well, and cultured by the use of RPMI 1640 (Gibco-BRL) containing 5% FBS and 10 µg/ml gentamicin (Sigma), in the presence of 5% CO₂ at 37 °C for 6 days. However, the invention is not limited to it.

M-CSF may be purchased, for example, fromPeproTechInc., but it may be produced in any ordinary genetic engineering technique by introducing M-CSF coding gene into a suitable protein expression vector. There is no specific limitation on M-CSF for use herein. It is desirable that M-CSF is selected in accordance with the species of the animal cells to be used herein, but M-CSF derived from any other different species is usable within an acceptable reactivity range.

More preferably, suitable cytokine and growth factor are added to the cells being cultured. The cytokine and growth factor to be added may be any ones that are reported effective for maintenance, growth promotion and differentiation induction of hematopoietic stem cells (Miller et al., *Proc. Natl. Acad. Sci. USA*, 94, 13648-13653, 1997; Matsunaga et al., *Blood,* 92, 452-461, 1998; Bryder et al., *Blood,* 96, 1748-1755, 2000), such as SCF, IL-3, IL-6, IL-11, G-CSF, GM-CSF, FLT, TPO, leukemia-inhibiting factor (LIF), basic fibroblast growth factor (basic FGF), hepatocyte growth factor (HGF), vascular endothelium growth factor (VEGF), insulin-like growth factor (IGF), to which, however, the invention should not be limited.

As so stated hereinabove, hematopoietic stem cells are defined as cells that have both the ability to produce all hemocytes (pluripotency) and the ability to self-renew themselves (self-renewal capability). Accordingly, for confirming whether hematopoietic stem cells could be expanded or not, a method is preferred capable of confirming the two abilities of the cells. For example, when mouse cells are used, they may be confirmed through long-term repopulation assay. This method will be described in more detail in the section of Example. Briefly, cells that contain hematopoietic stem cells are transplanted into an animal, and the animal is monitored as to whether or not the stem cell-derived blood cells exist for a long period of time in the blood of the animal. At present, this is the most reliable method for quantification of hematopoietic stem cells (Harrison et al., *Exp*. *Hematol*., 21, 206-219, 1993).

In case where human cells are used, applicable is a method of using NOD/SCID (non-obese diabetic/severe combined immunodeficiency disease) mice or β₂-microglobulin defective NOD/SCID mice as animals for transplantation (Larochelle et al., *Nat*. *Med*., 2, 1329-1337, 1996; Kollet et al., *Blood*, 95, 3102-3105, 2000).

The concentration of M-CSF to be added to the medium in cell culture is preferably from 0.1 to 100 ng/ml or so, more preferably from 1 to 50 ng/ml or so, most preferably from 5 to 20 ng/ml or so. The ability for expansion of hematopoietic stem cells was investigated by adding 0, 1, 10 or 100 ng/ml of M-CSF thereto and, as a result, it has been found that, when 10 ng/ml of M-CSF was added thereto, it significantly enhanced the cell expansion, but 100 ng/ml thereof rather retarded the cell expansion. This suggests the presence of an optimum concentration range of M-CSF for hematopoietic stem cell expansion.

Cells further selected from Lin-negative or weakly-positive cells by the positive selection of c-Kit and Sca-1 that are known as hematopoietic stem cell marker did not show hematopoietic stem cell expansion with M-CSF treatment. In Lin-negative or weakly-positive cells, hematopoietic stem cells are found in the M-CSF receptor (c-fms) -negative fraction. From this, it is considered that M-CSF would not directly act on hematopoietic stem cells but the effect of M-CSF on the hematopoietic stem cell expansion would be caused by an indirect action thereon via any other cells such as c-fms-positive cells or via some factor derived from the cells.

The invention further provides a kit containing M-CSF for culturing Lin-negative or weakly-positive cell. The culture kit contains M-CSF along with suitable cytokine, medium and so on, and is therefore usable for ex-vivo culture of Lin-negative or weakly-positive cells.

Furthermore, since M-CSF enhances the expansion of cells that contain hematopoietic stem cells, as so mentioned hereinabove, itmaybe used as an agent for enhancing the expansion of hematopoietic stem cells. The agent for enhancing hematopoietic stem cell expansion containing M-CSF may contain suitable cytokines and growth factors along with M-CSF. If the cell culture with such an agent for enhancing hematopoietic stem cell expansion added thereto enables sufficient *ex-vivo* expansion of hematopoietic stem cells, then cells that are expanded from a small amount of bone marrow *ex-vivo*, peripheral blood or cord blood could be used as a source of hematopoietic stem cells. Further, if direct *in-vivo* administration of M-CSF enables significant *in-vivo* expansion of hematopoietic stem cells, then even a small amount of hematopoietic stem cells would be enough in transplantation, and if so, cord blood transplantation could be applied to not only children but also adults.

### BRIEF DESCRIPTION OF THE DRAWINGS

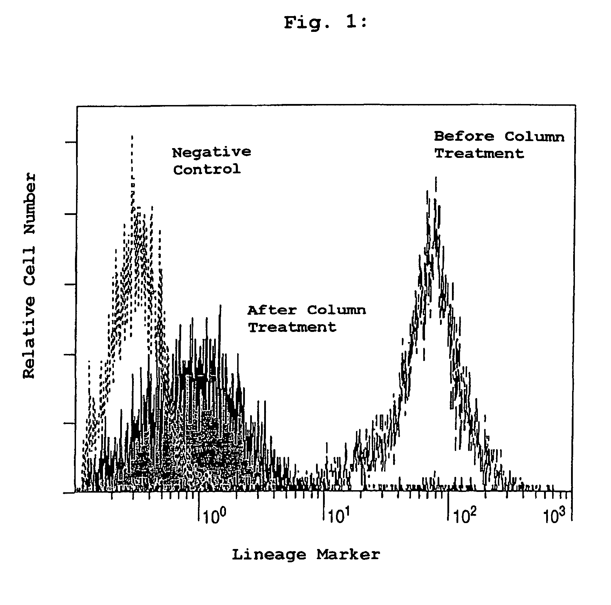

Fig. 1 shows flow cytometry charts of mouse bone marrow-derived cell populations labeled with anti-Lin antibody cocktail by the use of StemSep™. The chart "before column treatment" shows the analyzed data of the cell population not processed through a MACS LS⁺ column; and the chart "after column treatment" shows the analyzed data of the cell population processed through the column. It is understood that the treatment with StemSep™ gave lineage marker (Lin) -negative or weakly-positive cells.

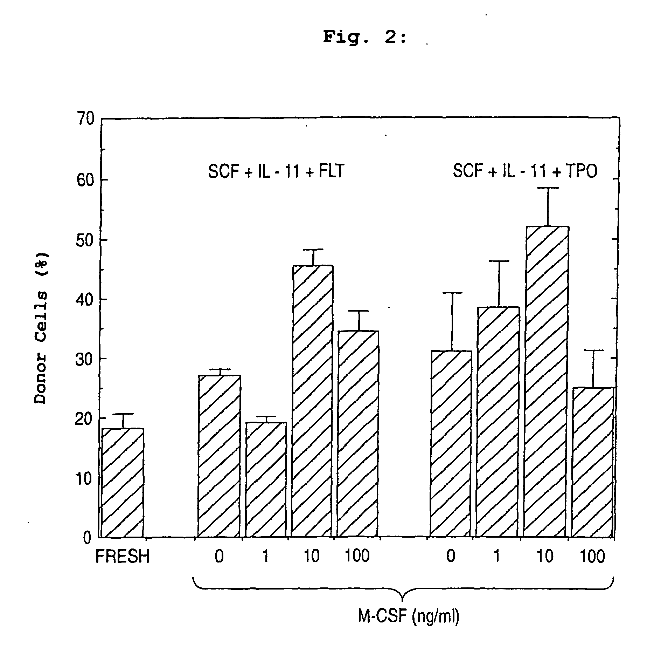

Fig. 2 shows cell expansion-enhancing activity of M-CSF. In this, the column of "percent donor cells" indicates the proportion of Ly5.1 mouse-derived cells in peripheral blood of Ly5.2 mice, for which Ly5.1 mouse-derived Lin-negative or weakly-positive cells were processed with various types of cytokine, and then transplanted into lethally irradiated Ly5.2 mice along with Ly5.2 mouse-derived cells, and the peripheral blood of the Ly5.2 mice was analyzed at 6 months after the transplantation. "Fresh" indicates no treatment with cytokine; "SCF + IL-11 + FLT" indicates cell culture with SCF, IL-11 and Flt-3 ligand along with M-CSF; and "SCF + IL-11 + TPO" indicates cell culture with SCF, IL-11 and TPO along with M-CSF.

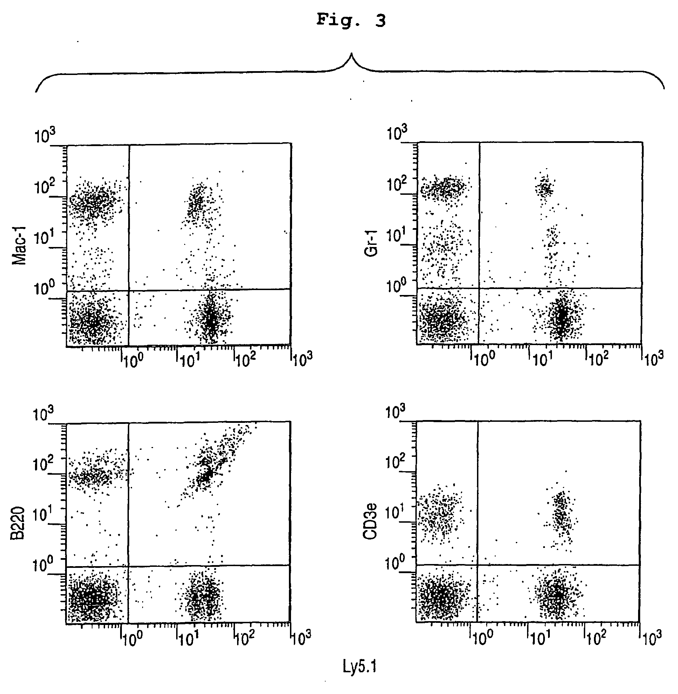

Fig. 3 shows expression of various differentiation markers of mouse peripheral blood leukocytes, for which Lin-negative or weakly-positive cells were cultured with SCF, IL-11 and Flt-3 ligand along with 10 ng/ml of M-CSF, the resulting cells were transplanted into mice and the mouse peripheral blood was analyzed at 6 months after the transplantation. Briefly, peripheral blood leukocytes were stained with biotinylated, various differentiation marker antibodies and PE-labeled streptoavidin, and then double-stained with FITC-labeled anti-mouse Ly5.1 antibody, and these were analyzed by flow cytometry. In the drawing, the vertical axis indicates the expression intensity of differentiation marker, Mac-1, Gr-1, B220 or CD3e; and the horizontal axis indicates the expression intensity of Ly5.1 (derived from transplanted cells).

### BEST MODE FOR CARRYING OUT THE INVENTION

The invention is described concretely with reference to the following Examples, to which, however, the invention should not be limited.

### Example 1:

### (Collection of marrow cells)

Marrow cells were collected from the femur and the tibia of a C57BL/6J-Ly5.1 mouse (Jackson Laboratories), suspended in a Hunks, balanced salt solution (Gibco-BRL) (hereinafter referred to as HBSS) containing 5% fetal bovine serum (FBS, Immunobiological Laboratory), and formed into a cell-floating suspension by the use of a 1-ml syringe equipped with a 22-gauge needle. This was filtered through a nylon mesh (Falcon) having a pore size of 40 µm to remove the cell aggregates. This was washed through centrifugation at 1,100 rpm for 5 minutes, and then again suspended in HBSS to be a single cell-floating suspension of the marrow cells.

### (Preparation of Lin-negative or weakly-positive cells)

Using StemSep™ (StemCell Technologies Inc.) for mouse hematopoietic precursor cell preparation, differentiation antigen-negative or weakly-positive (Lin < weakly-positive) cells were prepared according to the manual attached to the kit. First, a biotinylated antibody cocktail against differentiation antigens was added to the single cell suspension, and reacted on ice for 20 minutes, then washed with HBSS, and centrifuged at 1,100 rpm for 5 minutes. The cells were again suspended in HBSS, and a tetrameric antibody complex cocktail for binding of the biotinylated antibodies was added thereto and reacted on ice for 40 minutes, and then a magnetic colloid was added thereto and further reacted for 40 minutes. The cell suspension was passed through a MACS LS⁺ column (Miltenyi Biotec.) to make the labeled cells adsorbed, and the non-labeled cells were collected. PE (phycoerythrin)-labeled streptoavidin (Pharmingen) was added to a part of the collected cells, and this was analyzed with an EPICS-XL flow cytometer (Beckman Coulter). As shown in Fig. 1, the collected cells were Lin < weakly-positive, primitive cell populations.

### (Culture)

Thus obtained, the Lin < weakly-positive cells were seeded in a 6-well microplate for tissue culture (Iwaki) in an amount of 4 × 10⁴ cells/well, and cultured by the use of RPMI 1640 (Gibco-BRL) containing 5% FBS and 10 µg/ml gentamicin (Sigma) , in the presence of 5% CO₂ at 37 °C. SCF, IL-11, FLT and TPO were used as the cytokine to be added to the culture, and their concentration was 100 ng/ml. M-CSF was added thereto to confirm its effect, and its final concentration was 1, 10 or 100 ng/ml. All the cytokines used herein were purchased from Pepro Tech. Inc.

### (Transplantation of cultured cells into mice)

After cultured for 6 days in the presence of cytokines, the cells were collected from the microplate by pipetting. The cells were suspended in HBSS, and 10⁶ fresh bone marrow cells that had been prepared from C57BL/6J-Ly5.2 mice (Nippon Clea) were added thereto, and centrifuged at 1,100 rpm for 5 minutes. Finally, the cells were suspended in 400 µl of 1 mM HEPES-containing HBSS, and transplanted into lethally irradiated (800 roentgens) C57BL/6J-Ly5.2 mice through their tail vein.

### (Detection of transplanted cell-derived leukocytes)

Six months after the cell transplantation, about 25 µl of peripheral blood was collected from the tip of the tail of each mouse through a heparin-coated capillary, and then immediately suspended in 500 µl of phosphate buffer (PBS(-); Gibco-BRL) containing 10 units/ml of heparin. The supernatant was removed by centrifugation at 1,100 rpm for 5 minutes, and the cells were suspended in 500 µl of HBSS. Next, 10 µl of FITC-labeled anti-mouse Ly5.1 (Pharmingen) that had been 10-fold diluted with PBS (-) was added to it, and reacted on ice in the dark for 30 minutes. The cells were collected through centrifugation at 1,100 rpm for 5 minutes, and then suspended in 500 µl of a hemolytic solution comprising 155 mM ammonium chloride (NH₄Cl), 10 mM potassium bicarbonate (KHCO₃) and 0.1 mM ethylenediamine-tetraacetic acid (EDTA), at room temperature. The treatment for hemolysis was repeated twice. The cells were washed with HBSS, and collected through centrifugation at 1,100 rpm for 5 minutes, and then suspended in HBSS containing 2 µg/ml of 7-aminoactinomycin D, 0.2% bovine serum albumin, 0.05% sodium azide, and 0.02% ethylenediamine-tetraacetic acid (EDTA), and analyzed with an EPICS-XL flow cytometer.

As shown in Fig. 2, the proportion of the transplanted cell-derived leukocytes increased dependently on the M-CSF concentration. This may reflect increased self-renewal of hematopoietic stem cells during the culture period. However, a suppressive effect of M-CSF on the self-renewal of the cells was observed at higher concentrations, and it is understood that the effect of M-CSF on the enhancement of the self-renewal of hematopoietic stem cells is strictly controlled by its concentration (Fig. 2).

The results were quantified, as shown in Table 1. The activity of bone marrow reconstruction (repopulation unit, RU) equal to that of 10⁶ bone marrow cells is defined as 1 unit. Based on the calculation formula shown in the Table, the hematopoietic stem cell-activities before and after culture in the presence of cytokines were compared in point of the repopulation unit (RU). As RU was further increased more than two times by the addition of 10 ng/ml of M-CSF, it was experimentally confirmed that M-CSF is useful for expansion of hematopoietic stem cells.

If hematopoietic stem cells can be expanded at high degree as described above, it may be possible to reduce the necessary number of the stem cells to be collected for hematopoietic stem cell transplantation. To that effect, the present invention is useful from the viewpoint of ensuring the safety of donors.

**Table 1**

| Effect of M-CSF on Hematopoietic Stem Cell Expansion | | | |
|---|---|---|---|
| | %Ly5.1 | RU | Fold of Increase in Expansion |
| No Treatment | 18.4 ± 2.3 | 0.23 | 1.0 |
| SCF+IL-11+FLT | 27.2 ± 1.1 | 0.37 | 1.6 |
| SCF+IL-11+FLT+M-CSF | 45.5 ± 2.9 | 0.83 | 3.6 |
| SCF+IL-11+TPO | 31.2 ± 9.8 | 0.45 | 2.0 |
| SCF+IL-11+TPO+M-CSF | 52.2 ± 6.1 | 1.09 | 4.7 |
| RU = %Ly5.1/(100 - %Ly5.1) | | | |

### (Detection of differentiation antigen on transplanted cells-derived leukocytes)

Six months after the cell transplantation, about 100 µl of peripheral blood was collected from the tip of the tail of each mouse through a heparin-coated capillary, immediately suspended in 2 ml of phosphate buffer (PBS(-); Gibco-BRL) containing 10 units/ml of heparin, and then divided into 4 portions for staining with antibody. The cells were washed and then suspended in 500 µl of HBSS, 2 µl/tube of biontinylated anti-mouse Mac-1, anti-mouse Gr-1, anti-mouse B220 or anti-mouse CD3e (Pharmingen mouse linage panel) was added thereto as an antibody against differentiation antigen, and the antibody reaction was carried out on ice for 20 minutes. After the reaction, the cells were washed twice with HBSS, and again suspended in 500 µl of HBSS. Further, 0.5 µl of PE-labeled streptoavidin (Pharmingen) and 1 µl of FITC-labeled anti-mouse Ly5.1 (Pharmingen) were added, and reacted on ice in the dark for 20 minutes. Then, the cells were collected by centrifugation at 1,100 rpm for 5 minutes, and suspended in 500 µl of a hemolytic solution comprising 155 mM ammonium chloride (NH₄Cl), 10 mM potassium bicarbonate (KHCO₃) and 0.1 mM ethylenediamine-tetraacetic acid (EDTA), at room temperature. The treatment for hemolysis was repeated twice. The cells were washed with HBSS, and collected through centrifugation at 1,100 rpm for 5 minutes, and then suspended in HBSS containing 2 µg/ml of 7-aminoactinomycin D, 0.2% bovine serum albumin, 0.05% sodium azide, and 0.02% ethylenediamine-tetraacetic acid (EDTA), and analyzed with an EPICS-XL flow cytometer.

As so mentioned hereinabove, hematopoietic stem cells have both the ability of self-renewal and the ability to differentiate into all hemocytes. If no hematopoietic stem cells existed, the transplanted cells-derived hemocytes should have disappeared at about 3 months after the cell transplantation. As shown in Fig. 3, the transplanted cells-derived (Ly5.1 mouse bone marrow-derived) cells that express a differentiation antigen for myelocytes and lymphocytes were detected in the transplanted mice at 6 months after the transplantation. This confirms that the transplanted cell populations contained hematopoietic stem cells (Fig. 3).

While the invention has been described in detail and with reference to specific embodiments thereof, it will be apparent to one skilled in the art that various changes and modifications can be made therein without departing from the spirit and scope thereof.

The present application is based on a Japanese patent application filed on October 30, 2001 (Application Number 2001-331940), and its contents are incorporated herein for reference.

### INDUSTRIAL APPLICABILITY

The method of culture, the method of expansion, the hematopoietic stem cell populations obtained by the methods, the kit for culture and the agent for enhancing expansion of the invention enable *ex-vivo* expansion of hematopoietic stem cells, and are applicable to hematopoietic stem cell transplantation. Further, direct administration of the expanding agent enables cell transplantation with a reduced number of hematopoietic stem cells.

## Claims

1. Amethod for culturing Lin-negative or weakly-positive cells in the presence of macrophage colony-stimulating factor (M-CSF).

2. A method for expanding hematopoietic stem cells by culturing Lin-negative or weakly-positive cells in the presence of macrophage colony-stimulating factor (M-CSF).

3. A hematopoietic stem cell population obtained by the method of claim 2.

4. A kit for culturing Lin-negative or weakly-positive cells containing macrophage colony-stimulating factor (M-CSF).

5. An agent for enhancing expansion of hematopoietic stem cells containing macrophage colony-stimulating factor (M-CSF).
